# EUROPÄISCHE PATENTSCHRIFT

(11) **EP 0 728 440 B1**
(45) Veröffentlichungstag und Bekanntmachung des Hinweises auf die Patenterteilung: **04.10.2001**
(21) Anmeldenummer: 96102821.4
(22) Anmeldetag: 26.02.1996
(51) Int. Cl.: A61B 5/00, A61B 5/11

(54) **Auswerteverfahren und Vorrichtung zur tiefenselektiven, nicht-invasiven Detektion von Muskelaktivitäten**
Method and device for deep-selective, non-invasive detection of muscle activity
Procédé et appareil pour la détection non-invasive, profonde et sélective de l'activité musculaire

(30) Priorität: 24.02.1995 DE 19506484; 03.11.1995 DE 19541048
(43) Veröffentlichungstag der Anmeldung: 28.08.1996
(73) Patentinhaber: Ott, Lutz, 35463 Fernwald (DE)
(72) Erfinder: Ott, Lutz, 35463 Fernwald (DE); Steiner, Rudolf, Prof. Dr., Inst. für Lasertechn., 89081 Ulm (DE); Hülser, Paul Jürgen, Dr.med., 88353 Kisslegg (DE)
(74) Vertreter: Müller, Eckhard, Dr.

(56) Entgegenhaltungen:
- DE-A- 4 314 835
- US-A- 4 590 948
- AIZU Y ET AL: "BIO-SPECKLE PHENOMENA AND THEIR APPLICATION TO THE EVALUATION OF BLOOD FLOW" OPTICS AND LASER TECHNOLOGY, Bd. 23, Nr. 4, 1. August 1991, Seiten 205-219, XP000262901
- HON H ET AL: "DETECTION OF SKIN DISPLACEMENT AND CAPILLARY FLOW USING AN OPTICAL STETHOSCOPE" PROCEEDINGS OF THE NORTHEAST BIOENGINEERING CONFERENCE, NEWARK, MAR. 18 - 19, 1993, Nr. CONF. 19, 18. März 1993, Seite 189/190 XP000399711 LI J K-J;REISMAN S S
- DOUGHERTY G: "A LASER DOPPLER FLOWMETER USING VARIABLE COHERENCE TO EFFECT DEPTH DISCRIMINATION" REVIEW OF SCIENTIFIC INSTRUMENTS, Bd. 63, Nr. 5, 1. Mai 1992, Seiten 3220-3221, XP000301265

## Beschreibung

Die Erfindung betrifft eine Vorrichtung sowie ein Auswerteverfahren zur tiefenselektiven, nichtinvasiven Messung von Muskelaktivitäten in menschlichem, tierischem oder dergleichen biologischem Gewebe, wobei eine kohärente, monochromatische Lichtquelle zur Aussendung von Photonen hinein in das Gewebe durch einen örtlich definierten ersten Bereich des Gewebes oder der das Gewebe abdeckenden Haut vorgesehen ist, und mehrere Detektoren zur Erfassung der aus weiteren Flächenbereichen der Haut oder des Gewebes wieder austretenden Photonen vorgesehen sind, wobei die weiteren Flächenbereiche in unterschiedlichen Abständen von dem ersten Bereich angeordnet sind.

Seit langem wird die Elektromyographie in der Neurologie als wichtiges Instrument zur Untersuchung von Erkrankungen und Schäden im Bereich des peripheren Nervensystems sowie der Muskulatur eingesetzt. Eine in der täglichen klinischen Routine ganz wichtige Fragestellung besteht in der Diagnose des Ausmaßes einer eingetretenen Nervenschädigung. Einer Muskelschwäche sieht man in der Regel primär nicht an, ob ihr eine schwere, etwa einen operativen Eingriff erfordernde, Nervenschädigung oder aber lediglich ein vorübergehender Funktionsausfall des Nerven zugrunde liegt. Muskelfasern sind solange ausschließlich auf Nervenimpulse hin aktiv, solange dieser Nerv und die Verbindung Nerv-Muskelfaser intakt ist. Ist die zuständige Nervenfaser zerstört, beginnt die Muskelzelle spontan zu zucken, was elektrophysiologisch dem Auftreten sogenannter pathologischer Fibrillationen und positiver Wellen entspricht. Dieses Phänomen wird als Spontanaktivität bezeichnet.

Mit der bisher bekannten, konventionellen Elektromyographie überprüft man den betroffenen Muskel auf Auftreten der pathologischen Spontanaktivität. Der Nachweis erfolgt durch Einstich einer Nadelelektrode in den betroffenen Muskel. Allerdings muß an möglichst vielen Stellen eines Muskels oder mehrerer Muskeln eingestochen werden, um eine Differenzierung möglicher Ursachen einer Schädigung zu erhalten. Bei der Anwendung dieser konventionellen Elektromyographie kann es aufgrund des sehr schmerzhaften Einstechens der Nadelelektrode in den oder die Muskel zu Verkrampfungen kommen. Diese Verkrampfungen führen nicht selten zu Verfälschungen oder gar völlig unbrauchbaren Messungen. Besonders bei Kleinkindern und Säuglingen ist die sogenannte invasive Elektromyographie nur bedingt einsetzbar, da ein willentliches Entkrampfen des Patienten nicht erfolgt und somit eine eindeutige Messung nur sehr schwer durchführbar ist und die Meßergebnisse unter Umständen nicht eindeutig interpretierbar sind. Außerdem ist dieses invasive Verfahren mit einem Infektionsrisiko und einer starken Belastung des Patienten verbunden.

Andere zur Detektion von Fibrillationen bekannte Verfahren bedienen sich der Oberflächen-Elektromyographie unter Ableitung der Summenpotentiale. Diese Verfahren erlauben jedoch nicht den Nachweis spontaner Einzelkontraktionen, so daß keine ausreichende Differenzierung axonaler Läsionsformen möglich ist. Des weiteren sind diese Oberflächen-Elektromyographie-Verfahren der invasiven Elektromyograhie bei pathologischen Spontanaktivitäten unterlegen.

Eine Vorrichtung mit den eingangs genannten Merkmalen ist aus der DE 43 14 835 A1 bekannt und dient zur Bestimmung der Konzentration von Glukose in einer biologischen Matrix. Dabei sind mindestens zwei Detektionsmessungen vorgesehen, bei denen jeweils Licht als Primärlicht in die biologische Matrix eingestrahlt wird, das Licht in der biologischen Matrix entlang einem Lichtweg propagiert und eine als Sekundärlicht austretende Lichtintensität gemessen wird. Weiterhin ist eine Auswertung vorgesehen, bei der aus den Intensitätsmeßwerten der Detektionsmessung mittels eines Auswertealgorithmus und einer Kalibration die Glukosekonzentration abgeleitet wird. Eine tiefenselektive Bestimmung der Konzentration von Glukose ist mit dieser bekannten Vorrichtung nicht möglich.

Das Verfahren mit den eingangs genannten Merkmalen ist aus der US 4 590 948 bekannt. Dieses bekannte Verfahren dient zur Messung der Oberflächendurchblutung, wozu die aus dem Gewebe austretenden Photonen in zwei Flächenbereichen erfaßt werden, die äquidistant zum Eintrittsbereich angeordnet sind. Die Frequenz und Intensität der Photonen wird mittels Detektoren erfaßt, wobei jedoch auch bei diesem Verfahren eine tiefenselektive Flußdetektion nicht möglich ist.

Demgegenüber liegt der Erfindung die Aufgabe zugrunde, eine Vorrichtung sowie ein Verfahren zur nichtinvasiven, tiefenselektiven Detektion von Spontanaktivitäten und allgemeinen Muskelaktivitäten im Gewebe anzugeben.

Diese Aufgabe wird bzgl, der Vorrichtung i. w. dadurch gelöst, daß die austretenden Photonen mittels der Detektoren bezüglich Frequenz und Anzahl beziehungsweise Intensität detektiert werden und aus einem detektierten Interferenzmuster der aus dem jeweiligen Flächenbereich und deren Abstand zum Einstrahlort austretenden Photonen in Verbindung mit frequenzunverschobenen Photonen eine tiefenselektive Detektion von Muskelaktivitäten durchgeführt wird.

Das erfindungsgemäße Auswerteverfahren ist in Anspruch 12 definiert und hat die weitere merkmalen, daß die Flächenbereiche in unterschiedlichen Abständen von dem ersten Bereich angeordnet sind und man aus den Informationen Frequenz und Anzahl beziehungsweise Intensität in Verbindung mit dem jeweiligen Flächenbereich als Austrittsort der wieder austretenden Photonen mittels eines Auswerteprogramms oder -algorithmus tiefenselektive Informationen der Muskelaktivität und/oder Anzahl der aktiven Muskeln und/oder räumlichen Lage der aktiven Muskeln im Gewebe gewinnt.

Diese Vorrichtung bzw. dieses Auswerteverfahren nutzen die Eigenschaft der Wechselwirkung von elektromagnetischen Wellen an Gewebe und Muskelfasern aus. Aufgrund einer ausreichend kleinen Wellenlänge und dem damit verbunden, hinreichenden Verhältnis von Wellenlänge zu geometrischer Abmessung des zu detektierenden Körpers bzw. Gewebes, eignet sich der Spektralbereich von sichtbaren bis infraroten elektromagnetischen Wellen. Gleichzeitig wird eine ausreichende Detektionstiefe in biologischem Gewebe erreicht. Dieses Verfahren ist nicht invasiv und die aus den Wechselwirkungen entstehenden Änderungen sind.proportional zur Geschwindigkeit, der Anzahl und der Tiefe der einzelnen sich bewegenden Muskelfasern, so daß aus diesen Informationen eindeutig eine pathologische Spontanaktivität o. dgl. nachweisbar und zuweisbar ist. Untersuchungen sowohl im klinischen Alltag als auch bei speziellen, medizinischen Fragestellungen zur tiefenselektiven Detektion von Muskelaktivitäten lassen sich mit dieser Vorrichtung bzw. diesem Auswerteverfahren äußerst einfach, schmerzfrei und reproduzierbar durchführen.

Die Erfindung basiert i. w. darauf, mittels Laserlicht o. dgl., kohärente und monochromatische elektromagnetische Strahlung auf die Hautoberfläche bzw. Gewebeoberfläche der zu untersuchenden Stellen einzustrahlen. Die Photonen dringen in das Gewebe ein und werden entsprechend der optischen Parameter des Gewebe gestreut bzw. absorbiert. Da die Streuung mit einer Änderung der Ausbreitungsrichtung der Photonen einhergeht, werden auch Photonen aus dem Gewebe remittiert, d.h. an die Oberfläche des Gewebe bzw. der Haut zurückgestreut und treten wieder aus dem Gewebe aus. Diese Remission der aus dem Gewebe wieder austretenden Photonen weist eine abnehmende Intensität bei zunehmenden Abstand von dem Eintrittsort der Photonen auf. Ein weiteres Merkmal des biologischen Gewebes ist es, daß das Licht nicht gleichmäßig, d.h. isotrop, in alle Richtungen gestreut wird, sondern eine Vorwärtscharakteristik beim Streuprozeß erhalten bleibt. Das drückt sich in dem sogenannten Anisotropiefaktor g für Streuprozesse aus, der bei Gewebe einen Wert g ungefähr 0,9 annimmt. Ein Wert g = 0 würde isotroper, ein Wert g = 1 reiner Vorwärtsstreuung entsprechen.

Anhand eines einfaches Modell soll im folgenden erläutert werden, inwieweit über eine Detektion der remittierten Photonen ein Rückschluß auf den Zustand des Gewebes bzw. der Muskelfasern möglich ist: Betrachtet man z.B. Photonen, die etwa 5 mm neben dem Einstrahlort der Photonen aus dem Gewebe wieder heraustreten, dann kann mit hoher Wahrscheinlichkeit davon ausgegangen werden, daß sich diese wieder austretenden Photonen durch verschiedene Streuprozesse in etwa auf einer halbkreisförmigen oder ähnlichen Bahnkurve durch das Gewebe bewegt haben. Aufgrund der speziellen Anordnung der Meßvorrichtung bzw. der Durchführung des Auswerteverfahrens ist jedoch sicher, daß der Beginn der Bahnkurve am Eintrittsort der Photonen und das Ende der Bahnkurve am Meßpunkt der austretenden Photonen liegt. Sofern diese wieder austretenden Photonen überhaupt eine Information bzgl. der Bewegung einzelner Muskelfasern o. dgl. haben sollten, kann jedenfalls davon ausgegangen werden, daß die direkt neben dem Einstrahlort wieder aus dem Gewebe austretenden Photonen nur Informationen bzgl. dicht unterhalb der Oberfläche angeordneter Muskelfasern tragen, während solche in weiterem Abstand von dem Einstrahlort austretende Photonen Aufschluß auch über tiefere Gewebeschichten geben können. Diese Modellbetrachtung verdeutlicht somit, daß durch eine Detektion von aus dem Gewebe wieder austretenden Photonen mit zunehmendem Abstand vom Einstrahlort selektiv Informationen aus bestimmten Gewebetiefen erhalten werden können.

Zur Messung bewegter, streuender Teilchen wird bekanntermaßen der optische Doppler-Effekt herangezogen. Dabei erfährt Licht beim Streuprozeß eine Frequenzverschiebung, die proportional zur Geschwindigkeit des bewegten Teilchens zunimmt. Unter Berücksichtigung des Doppler-Effektes kann so z.B. in oberflächlichen Gewebeschichten die Muskelaktivität im Gewebe bestimmt werden. Um ein optimales Doppler-Signal von fibrillierenden Muskelfasern in tieferen Gewebeschichten zu erhalten, ist eine Lichtwellenlänge der Lichtquelle, insbesondere des Lasers, erforderlich, die vom Gewebe nur wenig absorbiert wird. Es bieten sich daher Wellenlängen im Bereich von etwa 600 nm bis etwa 1200 nm, bevorzugt bei etwa 820 nm an.

In der Literatur wird hin und wieder diskutiert, daß kohärentes Laserlicht beim Einstrahlen in das Gewebe durch die Vielzahl der Streuprozesse seine Kohärenzeigenschaften verlieren könnte. Durch interferometrische Untersuchungen läßt sich jedoch zeigen, daß ein gewisser Anteil von Photonen, die im größeren Abstand vom Einstrahlort aus dem Gewebe austreten, mit dem einfallenden Photonenstrahl interferieren kann, woraus zweifellos gefolgert werden muß, daß die Kohärenzeigenschaften dieser gestreuten Photonen noch vorhanden sind. Somit ist es aber auch möglich, in größeren Abständen von dem Einstrahlort noch Doppler-Signale mit der entsprechenden Frequenzverschiebung der wieder austretenden Photonen zu detektieren. Dabei mischen sich die Photonen mit Dopplerverschobener Frequenz mit solchen Photonen, die keine Doppler-Verschiebung erfahren haben, also nur von einer starren bzw. unbeweglichen Matrix gestreut wurden. Am Detektionsort an der Gewebeoberfläche entsteht somit eine Intensitätsschwebung zwischen frequenzverschobenen und nicht-frequenzverschobenen Photonen. Dies führt zu einem lokalen Speckle-Muster, dessen Intensität mit der Doppler-Frequenz variiert und somit von einem optischen Detektor gemessen werden kann.

Im Prinzip erfolgt die Auswertung der Signale in der Form, daß zwei Detektoren, bevorzugt symmetrisch zum Einstrahlort die remittierten Photonen erfassen. Die Ausgangssignale dieser Detektoren werden entsprechend ausgewertet und verarbeitet, um zu der gewünschten Information bzw. Aussage hinsichtlich der Muskelaktivitäten zu gelangen.

Zusammenfassend kann festgestellt werden, daß ein derartiges Auswerteverfahren bzw. eine derartige Vorrichtung besonders im Bereich des "therapeutischen Fensters", also bei Wellenlängen im Bereich von 600 nm bis 1200 nm, in dem die Streuung der eingestrahlten Photonen nicht vernachlässigbar ist, durchführbar ist. Die optische Absorption aus Streuung von Licht in menschlichem Gewebe kann durch die Photonentransporttheorie näher beschrieben werden. Hierbei wird der Pfad eines in die Haut eingestreuten Photons verfolgt. Das Photon erfährt an den einzelnen lokalen Streuern entweder eine elastische Streuung oder es wird vollständig absorbiert. Daraus läßt sich für Laserlicht des roten Wellenlängenbereichs (600 nm) bzw. des infraroten Wellenlängenbereichs (1200 nm) die Eindringtiefe und der Streuprozeß bestimmen. Obwohl die sogenannte mittlere freie Weglänge relativ kurz ist, kann Licht dieses Wellenlängenbereichs tief in das Gewebe eindringen, da die Streuung hauptsächlich in Vorwärtsrichtung erfolgt (sogenannte Mie-Streuung), die Streuvorgänge wesentlich häufiger sind als die der Absorption und die Absorption im Gewebe für diesen Wellenlängenbereich gering ist gegenüber anderen Wellenlängen. Die Lichtausbreitung im Gewebe wird nach der Transporttheorie durch folgende Parameter beschrieben: Anisotropiefaktor, Streukoeffizient, Absorptionskoeffizient, mittlere freie Weglänge.

Eine Gesamtschau der durchgeführten theoretischen wie auch experimentellen Untersuchungen deutet darauf hin, daß mit dem erfindungsgemäßen Verfahren bzw. der erfindungsgemäßen Vorrichtung ringförmige Interferenzstrukturen in konzentrischer Lage bzgl. des Einstrahlortes erhältlich sind. Mit zunehmendem lateralen Abstand zum Einstrahlpunkt legen die Photonen mit hoher Wahrscheinlichkeit im Gewebe größere Wege zurück und dringen dementsprechend auch tiefer in das Gewebe ein. Um die im Photonenzustand enthaltene Information über Bewegungen des ausgeleuchteten Gewebes auswerten zu können, sollte die Lichtquelle spezifische Eigenschaften, wie eine ausreichende Kohärenzlänge besitzen, monochromatisch sein und im Single-Mode-Zustand betreibbar sein.

Eine wichtige Voraussetzung zum Erhalt der gewünschten Information ist eine ausreichend hohe Kohärenzlänge, so daß ein Interferenzmuster auf der Oberfläche der erfindungsgemäßen Vorrichtung erzielbar ist. Das Entstehen des Interferenzmusters ist auf die Annahme zurückzuführen, daß Photonen, die nahe der Gewebe- bzw. Hautoberfläche gestreut werden, keine Frequenzveränderungen erfahren, wobei diese sozusagen nicht gestreuten Photonen mit solchen Photonen, die in der Tiefe an bewegten Teilchen, also an den Muskelfasern gestreut werden und dadurch eine Frequenzveränderung erfahren, interferieren. Wird daher das frequenzverschobene Streulicht, welches an sich bewegenden Teilchen gestreut wurde, mit frequenzunverschobenem quasi Originallicht, auf der Detektorfläche zur Deckung gebracht, entsteht eine Schwebungsfrequenz bzw. ein Interferenzmuster. Um diese Schwebungsfrequenzen aus tieferen Gewebeschichten zu erhalten, ist der Einsatz einer Wellenlänge im Bereich von 600 nm bis ca. 1200 nm erforderlich, wobei ebenfalls auf eine ausreichende Kohärenzlänge der Lichtquelle geachtet werden sollte. Es konnte gezeigt werden, daß typische Interferenzmuster auch für große Gewebetiefen nachweisbar sind und daß die Informationen mit zunehmendem Abstand der Detektorfläche aus zunehmender Tiefe des Gewebes stammt. Dieser Nachweis ist auch für große laterale Abstände der Detektorfläche vom Einstrahlort in einem Bereich von bis zu ca. 15 mm erfolgreich durchgeführt worden. Allerdings erscheinen auch laterale Abstände bis 30 mm in der Praxis grundsätzlich möglich zu sein.

Die erfindungsgemäße Vorrichtung weist eine Reihe von vorteilhaften Ausgestaltungen auf.

So ist es bspw. gemäß einer ersten vorteilhaften Ausführungsform der erfindungsgemäßen Vorrichtung möglich, daß der Lichtquelle wenigstens ein dem Gewebe stirnseitig aufbringbares oder aufsetzbares Lichtleitfaserstück, bzw. eine Kollimationsoptik zur Fokussierung des Laserlichts auf dem Gewebe bzw. der Haut, nachgeordnet ist. Durch diese Maßnahme wird eine örtlich definierte Einstrahlung der Photonen in das Gewebe gewährleistet. Das Lichtleitfaserstück sollte vorzugsweise eine Mono-Mode-Faser mit einem Durchmesser von ca. 300 um und größer sein, um die Lichtleistung es Halbleiterlasers übertragen zu können. Es können allerdings auch Multi-Mode-Fasern eingesetzt werden. Je nach Ausführung kann auch auf das Lichtleitfaserstück verzichtet werden, bspw. dann, wenn das Laserlicht mittels einer Kollimationsoptik auf der Hautoberfläche fokussiert wird.

Weiterhin hat es sich als äußerst vorteilhaft erwiesen, daß die Detektoren jeweils eine dem Gewebe stirnseitig aufbring- oder aufsetzbare Lichtfaser aufweisen, denen eine Photodiode o. dgl. nachgeordnet ist. Somit besteht die Detektorfläche aus an den Stirnseiten polierten Glasfaserstücken, deren räumliche Lichtstromverteilung so ausgelegt ist, daß das empfangene Licht jeweils auf die sensitive Fläche einer Photodiode weitergeleitet wird.

Zur Verbesserung der Signalgüte hat sich der Einsatz eines der Lichtquelle nachgeschalteten bzw. den Detektoren vorgeschalteten Polarisationsfilters erwiesen, wobei den Detektoren ggf. zusätzlich ein Absorptionsfilter zugeordnet sein kann. Diese Filter können entweder aufgedampft oder auch als separate Glaseinlage ausgebildet sein.

Vorteilhafte Wellenlängen der bevorzugt als Halbleiterlaser ausgebildeten Lichtquelle liegen im Bereich von 600 nm bis etwa 1200 nm, bevorzugt bei etwa 820 nm. Dieser Wellenlängenbereich wird auch als "therapeutisches Fenster" bezeichnet.

Als eine ganz besonders vorteilhafte, eigenständige Ausgestaltung der Erfindung erweist sich die Maßnahme, daß die weiteren Flächenbereiche bzw. die zugeordneten Detektoren paarweise einander benachbart und i. w. in Reihe liegend hintereinander angeordnet sind, wobei jedes Paar einen anderen Abstand zum ersten Bereich aufweist und die Abstände benachbarter Paare insbesondere äquidistant sind. Somit sind jeweils zwei Detektorflächen vorgesehen, die paarweise nebeneinander und symmetrisch zum Einstrahlort der Photonen in einem definierten Abstand hiervon angeordnet sind. Jeder der paarigen Flächenbereiche bzw. Detektoren repräsentiert somit ein Meßvolumen des zu analysierenden Gewebes. Jeder dieser paarigen Detektoren sollte aufgrund der Lichtverteilung im Gewebe ein äquivalentes Signal aufnehmen.

Nach einer anderen Ausführungsform der Erfindung besteht auch die Möglichkeit, daß die weiteren Flächenbereiche bzw. die zugeordneten Detektoren auf eine durch den ersten Bereich laufenden Geraden und paarweise symmetrisch beidseitig des ersten Bereichs angeordnet sind. Allerdings kann bei dieser Ausführungsform unter Umständen das Problem auftreten, daß ausgehend von einer Vorzugsrichtung der Lichtausbreitung im Gewebe durch die paarigen Detektoren bzw. Flächenbereiche unterschiedliche Doppler-Frequenzen der wieder austretenden Photonen erfaßt und somit ein falsches Meßergebnis geliefert werden.

Im Prinzip ist es natürlich auch möglich, lediglich einen einzigen weiteren Flächenbereich bzw. Detektor pro Detektionskanal einzusetzen. Allerdings erweist sich in diesem Fall die Meßwerterfassung aufgrund eines ungünstigeren Signal-Rausch-Verhältnisses als erheblich aufwendiger.

Wie die praktischen Untersuchungen gezeigt haben, sind die weiteren Flächenbereiche bzw. die entsprechenden Detektoren zur Erfassung der wieder aus dem Gewebe austretenden Photonen bis zu einem maximalen Abstand von etwa 15 bis 30 mm von dem ersten Bereich angeordnet. Dieser maximale Bereich ist i. w. von der verwendeten Wellenlänge und dem damit einhergehenden Absorptionskoeffizient sowie von der Kohärenzlänge der verwendeten Lichtquelle abhängig.

Als besonders vorteilhaft hat es sich hinsichtlich der Auswertung der Signale der Detektoren erwiesen, daß die von paarweisen Flächenbereichen bzw. von den entsprechenden Detektoren erfaßten beiden Signale, jeweils den beiden Eingängen eines Differenzverstärkers zugeführt werden. Der Einsatz eines Differenzverstärkers bringt den Vorteil, daß der statische Signalanteil der paarig geschaltete Detektoren aufgrund der Verstärkung lediglich der an den Eingängen anliegenden Eingangsspannungsdifferenz kompensiert wird und somit i. w. nur die dynamischen Intensitätsfluktuationen, welche durch den optischen Doppler-Effekt und somit die bewegten Gewebeteile bedingt sind, verstärkt werden. Diese Maßnahme führt zu einer erheblichen Verbesserung des Signal-Rausch-Verhältnisses und somit zu einer erheblich genaueren Signalauswertung, da lediglich die auf Bewegungen des Gewebes zurückführenden Intensitätsschwankungen verstärkt werden.

Dadurch, daß die Signale der Detektoren, ggf. nach einer Digitalisierung, unter anderem einer adaptiven Filterfunktion sowie einer Cepstrumanalysefunktion unterworfen werden, bleibt zum einen das Nutzsignal aufgrund der Filterung unbeeinflußt und können zum anderen charakteristische, sich verändernde Frequenzanteile der Signale sichtbar gemacht werden.

Nach einem weiteren, vorteilhaften Aspekt der Erfindung sind die Lichtquelle sowie die Detektoren und elektronische Komponenten, wie ggf. Vorverstärker, Differenzverstärker und ggf. Analog/Digital-Wandler gemeinsam in einem Meßkopf, der insbesondere flächig auf das Gewebe bzw. die Haut auflegbar ist, untergebracht, wobei der Meßkopf lediglich mittels elektrischer Leiter mit der Auswerteeinheit, insbesondere dem Prozessor, verbindbar ist. Dieser Meßkopf kann bspw. ca. 130 mm hoch und ca. 15 mm breit und mit abgerundeten Seitenfläche ausgebildet sein. Jedoch sind auch andere Gehäuseausführungen je nach den individuellen Erfordernissen denkbar. Die gesamte Vorrichtung mit Ausnahme des Prozessors ist somit auf einer einzigen Trägerplatte oder Platine o. dgl. angebracht, die in einem geschlossen, physiologisch unbedenklichen und geschirmten Kunststoffgehäuse aufgenommen ist.

Eine besonders vorteilhafte Ausgestaltung des eingangs geschilderten, erfindungsgemäßen Auswerteverfahrens besteht darin, daß man eine Lichtquelle mit großer Kohärenzlänge, insbesondere größer als 10 cm, verwendet und man die wieder austretenden Photonen jeweils in einem bestimmten Abstand von dem ersten Bereich, insbesondere zeitgleich wenigstens in zwei dicht benachbarten oder symmetrisch zum ersten Bereich angeordneten Flächenbereichen, detektiert. Durch diese Maßnahme wird die Voraussetzung für ein besonders gutes Signal-/Rausch-Verhältnis geschaffen.

Dabei hat es sich als vorteilhaft erwiesen, daß man die detektierten Signale jeweils zweier Flächenbereiche unter anderem einer Differenzverstärkerfunktion zuführt sowie ggf. einer adaptiven Filterfunktion und ggf. einer Frequenz- und/oder Cepstrumanalayse unterwirft. Durch diese Art der verfahrensmäßigen Signalauswertung kann eine genaue Analyse der Muskelaktivitäten erhalten werden.

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1: eine schematische Ansicht eines ersten Ausführungsbeispiels der erfindungsgemäßen, auf ein Gewebe aufgesetzten Vorrichtung in Seitenansicht,
- Figur 2: ein zweites Ausführungsbeispiel der erfindungsgemäßen Vorrichtung in Seiten- und Unteransicht,
- Figur 3: ein drittes Ausführungsbeispiel der erfindungsgemäßen Vorrichtung in schematischer Darstellung,
- Figur 4: ein Blockdiagramm einer adaptiven Filterfunktion und
- Figur 5: ein Blockschaltbild einer Cepstrumanalysefunktion.

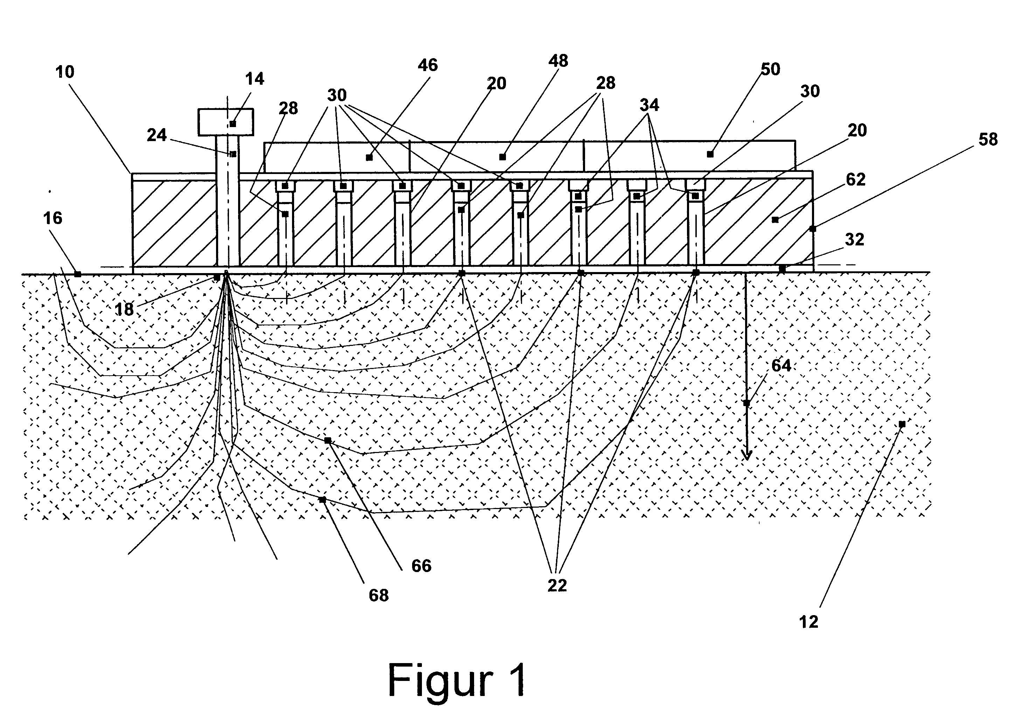

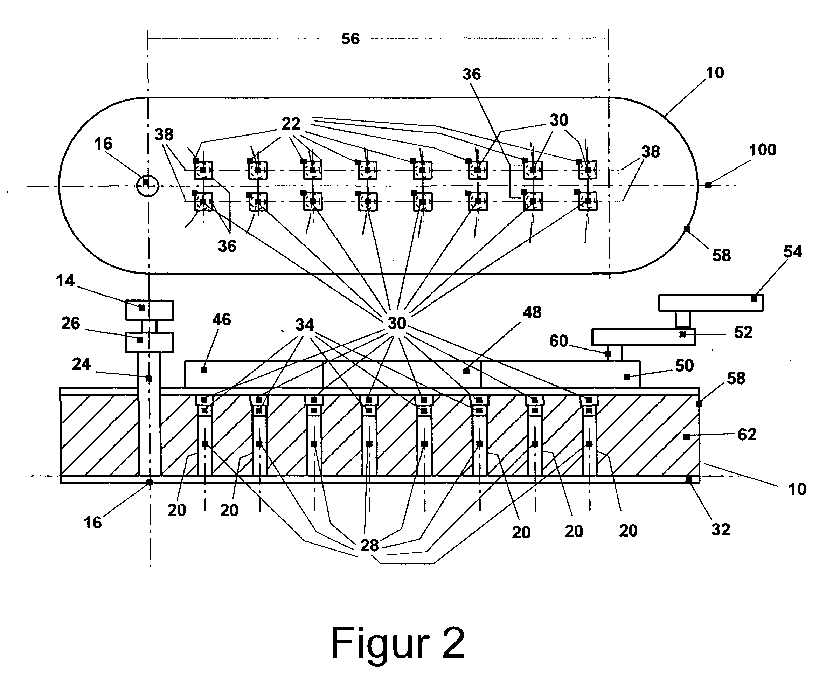

Die in den Figuren 1 und 2 dargestellte Vorrichtung 10 zur Messung von Muskelaktivitäten in menschlichem, tierischem o. dgl. Gewebe 12 weist eine Lichtquelle 14, insbesondere einen Halbleiterlaser, zur Aussendung von Photonen hinein in das Gewebe 12 auf. Die Vorrichtung 10 ist mit einer Trägerplatte auf das Gewebe 12 bzw. die Haut 18 des Gewebes aufgesetzt, wobei Photonen durch einen ersten Bereich 16, der örtlich i. w. wohl definiert ist, in das Gewebe 12 eintreten. Die Photonen werden in dem Gewebe 12 zum Teil gestreut, zum Teil absorbiert, wobei einige mögliche Photonenwegstrecken 66, 68 bildlich und schematisch in Figur 1 dargestellt sind. Deutlich ist sichtbar, daß die Photonen mit wachsender Eindringtiefe 64 immer weiter entfernt von dem ersten Bereich 16 aus dem Gewebe 12 wieder austreten.

Weiterhin weist die Vorrichtung 10 mehrere Detektoren 20 zur Erfassung der aus weiteren Flächenbereichen 22 der Haut 18 bzw. des Gewebes 12 austretenden Photonen auf. Die weiteren Flächenbereiche 22 sind in unterschiedlichen Abständen von dem ersten Bereich 16 beabstandet.

Der Lichtquelle 14 ist gemäß dem Ausführungsbeispiel der Figur 1 ein dem Gewebe 12 stirnseitiges aufsetzbares Lichtleitfaserstück 24 zugeordnet. Aus dem Ausführungsbeispiel der Figur 2 ist ersichtlich, daß jedoch anstelle des Leichtleitfaserstücks 24 auch eine Kollimationsoptik 26 zur Fokussierung des Laserlichts auf dem Gewebe 12 bzw. der Haut 18 zum Einsatz kommen kann.

Die Detektoren 20 weisen jeweils eine dem Gewebe 12 stirnseitig aufsetzbare Lichtleitfaser 28 auf, denen eine Photodiode 30 o. dgl. nachgeordnet ist.

Der Lichtquelle 14 ist ein Polarisationsfilter nach- und den Detektoren 20 vorgeschaltet. Weiterhin sind zwischen die Lichtleitfaser 28 und die Photodiode 30 ggf. Absorptionsfilter 34 eingebracht.

Die Wellenlänge des Halbleiterlasers liegt im Bereich von etwa 600 nm bis etwa 1200 nm, bevorzugt bei etwa 820 nm.

Wie insbesondere aus dem Ausführungsbeispiel der Figur 2 ersichtlich ist, sind die weiteren Flächenbereiche 22 bzw. die zugeordneten Detektoren 20 paarweise einander benachbart und i. w. in Reihe 38 liegend hintereinander angeordnet, wobei jedes Paar 36 einen anderen Abstand zum ersten Bereich 16 aufweist und die Abstände benachbarter Paare 36 jedenfalls im Ausführungsbeispiel äquidistant sind. Es versteht sich, daß auch andere Abstände der einzelnen Detektoren 20 bzgl. des ersten Bereichs 16 gewählt werden können, dies bemißt sich anhand der speziellen Erfordernisse des jeweiligen Systems und anhand des Fachwissens des Durchschnittsfachmanns. Die weiteren Flächenbereiche 22 bzw. die entsprechenden Detektoren 20 sind bis zu einem maximalen Abstand 56 von etwa 15 bis 30 mm von dem ersten Bereich 16 angeordnet.

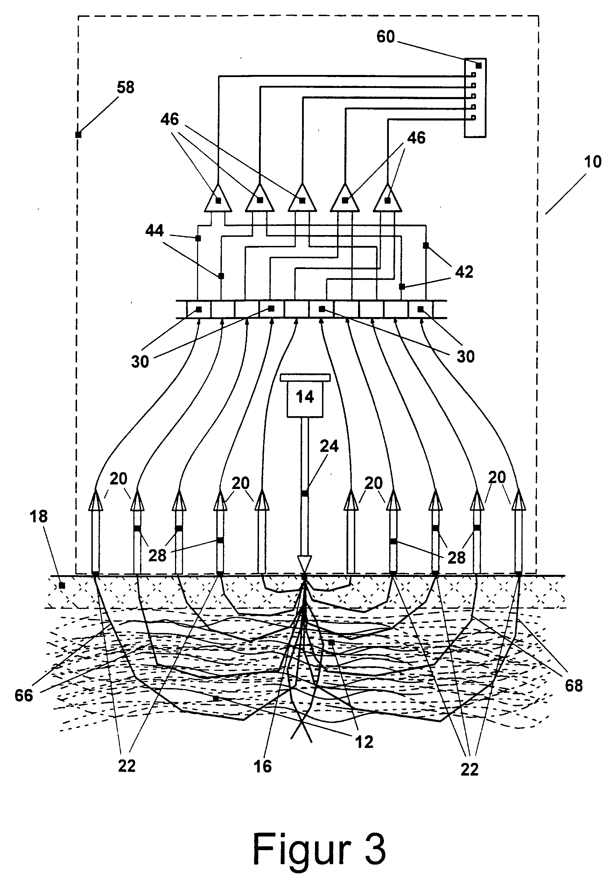

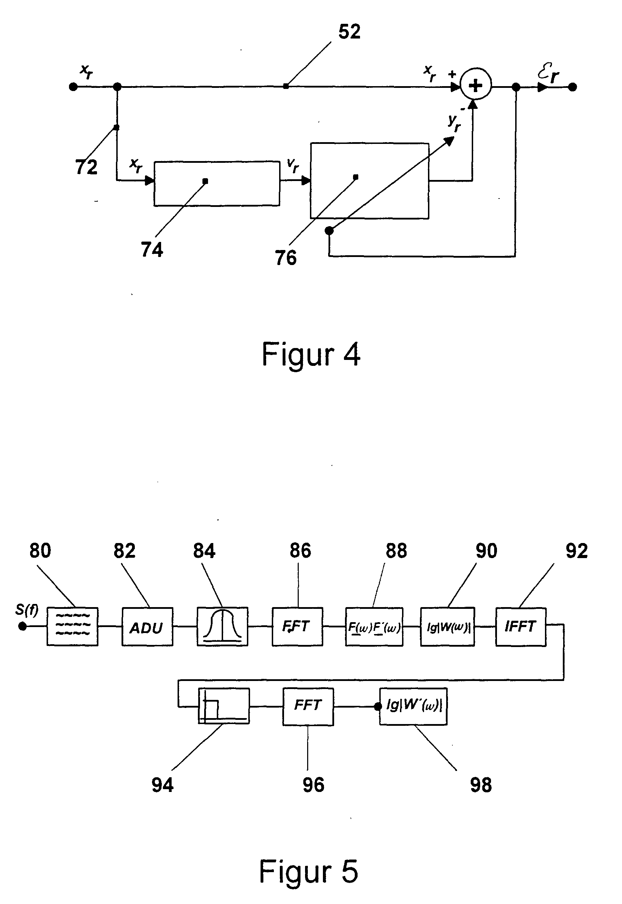

Wie insbesondere aus Figur 3 ersichtlich ist, werden die von paarweisen Flächenbereichen 22 bzw. von den entsprechenden Detektoren 20 erfaßten beiden Signale 42, 44 jeweils den beiden Eingängen eines Differenzverstärkers 46 zugeführt. Diese Maßnahme findet vorteilhafterweise auch von Vorteil bei den Ausführungsbeispielen der Figuren 1 und 2 Anwendung, wobei dann entsprechende Paare 36 von Detektoren an einen Differenzverstärker 46 angeschlossen sind. Die Signale der Detektoren 20 werden unter anderem einer adaptiven Filterfunktion 52 sowie einer Cepstrumanalysefunktion 78 unterworfen (Figur 5).

Die gesamte Vorrichtung mit Ausnahme eines Prozessors 54, also die Lichtquelle 14, die Detektoren 20 und die elektronischen Komponenten, wie ggf. Vorverstärker 48, Differenzverstärker 46 sowie Analog/Digital-Wandler 50 sind gemeinsam in einem Meßkopf 58 untergebracht, der flächig auf das Gewebe 12 bzw. die Haut 18 auflegbar ist. Der Meßkopf 58 weist somit lediglich eine Verbindung mittels elektrischer Leiter zu der Auswerteeinheit, insbesondere dem Prozessor 54 auf. Das Innere des Meßkopfes ist mit einer Füllmasse 62 ausgefüllt.

In Abweichung zu der Anordnung der Detektoren 20 der Ausführungsformen der Figur 1 und 2 weist die Ausführungsform gemäß der Figur 3 weitere Flächenbereiche 22 bzw. zugeordnete Detektoren 20 auf, die i. w. auf einer durch den ersten Bereich 16 laufenden Geraden 40 und paarweise symmetrisch beidseitig des ersten Bereichs 16 angeordnet sind.

Die adaptive Filterfunktion 52 in Figur 4 weist zwei Kanäle 70, 72 auf, wobei der erste Kanal 70 das unveränderte Eingangssignal führt, in dem Kanal 72 jedoch eine Verzögerungsstufe 74 eingeschaltet ist. Mittels der Adaptionsstufe 76 werden die Filterkoeffizienten solange geändert, bis die Differenz zwischen dem ungefilterten Eingangssignal des Kanals 70 und des gefilterten Signals des Kanals 72, im quadratischen Mittel minimal wird.

Gemäß Figur 5, welche ein Blockschaltbild der Cepstrumanalysefunktion 78 wiedergibt, werden die Signale als Zeit-Amplituden-Funktion graphisch dargestellt und mit einer reellwertigen Fast-Hartley-Transformation (FHT) bzw. einer Fast-Fourier-Transformation (FFT) oder einem anderen Frequenzanalysealgorithmus in ein Leistungsspektrum 88 bzw. Powerspektrum zerlegt. Hierbei werden die in der Schwebungsfrequenz enthaltenen Teilfrequenzen hinsichtlich ihrer Intensität und Bandbreite analysiert. Das Leistungsspektrum 88 wird in Echtzeit mit einer Stützpunktzahl größer 64 Punkte ermittelt und graphisch für jeden A/D-Wandler-Kanal 82 in eine Art "Wasserfalldarstellung" aufbereitet. Dieses Leistungsspektrum wird einer Momentenanalyse sowohl für die Intensität als auch für die Frequenz unterworfen. Die so gebildeten Momente lassen sich zu einem Vektor zusammenfassen und graphisch darstellen. Insgesamt besteht die Cepstrumanalysefunktion 78 aus der Hintereinanderschaltung einer Filterfunktion 80, eines A-/D-Umsetzers 82, eines diskreten Zeitfensters 84, einer Fast-Fourier-Transformation 86, einem Leistungsspektrum 88, einer Logarithmierfunktion 90, einer inversen Fast-Fourier-Transformation 92, einer Lifterfunktion 94, einer Fast-Fourier-Transformation 96 und schließlich dem modifizierten Leistungsspektrum 89. Dieser letztere Vorgang wird auch als Cepstrumanalysefunktion 78 bezeichnet, wodurch charakteristische, sich verändernde Frequenzen sichtbar gemacht werden können. Die Bedeutung der Cepstrumanalysefunktion liegt insbesondere darin, daß Spektren mit periodischen Schwankungen einer genauen Analyse unterzogen werden können. Das Cepstrum wird aus dem phasenlosen Leistungsspektrum gebildet.

Alle Meßdaten, Auswertungen und Analysen können auf einer graphischen Benutzeroberfläche dem Anwender visualisiert und zur weiteren Bearbeitung auf Massenspeichern archiviert werden. Die verschiedenen Signale der einzelnen Kanäle können auch weiterführend mit einer Kreuzkorrelation, der Autokorrelation und anderen Signalauswertealgorithmen miteinander verglichen und auswertet werden.

Die Ausführungsform der Figuren 1 und 2 funktioniert i. w. wie folgt. Ein in dem Meßkopf 58 angeordneter Halbleiterlaser strahlt kohärentes, monochromatisches Licht, insbesondere einer Kohärenzlänge größer als 10 cm, direkt in das Lichtleitfaserstück 24 ein. Das Licht wird in dem Lichtleitfaserstück 24 zur Oberfläche der Haut 18 in dem ersten Bereich 16 weitergeleitet. Das Lichtleitfaserstück 24 sollte vorzugsweise eine Mono-Mode-Faser mit einem Mindestdurchmesser von ca. 300 µm sein, um die Lichtleistung des Halbleiterlasers übertragen zu können. Je nach Ausführung kann auf das Lichtleitfaserstück 24 verzichtet werden, wenn das Laserlicht mit Hilfe einer Kollimationsoptik 26 unmittelbar auf die Hautoberfläche fokussiert wird. Das remittierte Licht wird an den Lichtleitfasern 28 in den weiteren Flächenbereichen 22 aufgenommen und zu den Photodioden 30 weitergeleitet. Vor oder hinter den Lichtleitfasern 28 und/oder vor dem Lichtleitfaserstück 24 sitzen jeweils Absorptionsfilter 34 bzw. Polariationsfilter 32.

Alle Fasern und Filter sind bevorzugt in einer lichtundurchlässigen Kunststoff- oder Keramikfassung auf dem Meßkopf 58 arretiert. Die Fasern sind paarweise außermittig einer Längsachse 100 auf einer Trägerplatte des Meßkopfes 58 angeordnet. Der Abstand zwischen den einzelnen Paaren 36 ist gleich, wobei dieser Abstand in verschiedenen Ausführungen variabel gestaltet werden kann. Die Lichtleitfasern 28 sollten einen Durchmesser von 400 µm nicht überschreiten, da das Signal-/Rauschverhältnis mit kleiner werdendem Durchmesser besser wird. Allerdings nimmt auch mit kleiner werdendem Durchmesser die Intensität des Meßsignals ab, so daß hier ein Kompromiß zu finden ist. Bei der Wahl der Photodioden 30 ist bevorzugt darauf zu achten, daß die einzelnen Photoströme bei gleicher Ausleuchtung nur minimal voneinander abweichen. Die gesamte Auswerteelektronik bestehend aus Vorverstärker 48 und Differenzverstärker 46 ist in dem abgeschirmten Gehäuse des Meßkopfs 58 integriert. Auch die Analog-/Digital-Wandler 50 können im Meßkopf 58 angeordnet sein. Allerdings besteht auch die Möglichkeit, diese Komponenten außerhalb des Meßkopfes 58 auf einer Meßkarte zu plazieren, so daß nur digitalisierte Signale an den Prozessor 54 weitergegeben werden. Die Auswerteelektronik hat die Aufgabe, den von den Photodioden 30 kommenden Strom in eine Spannung zu wandeln und zu verstärken, was mittels des Vorverstärkers 48 durchgeführt wird. Anschließend wird von paarweise angeordneten und geschalteten Photodioden 30 mittels der Differenzverstärker 46 eine Signaldifferenz gebildet und weiter verstärkt. Schließlich ist jedem Differenzverstärker 46 ein Analog-/Digital-Wandler 50 mit 12 bis 16 bit Auflösung und einer Mindestabtastrate von etwa 20 kHz nachgeschaltet. Somit wird jedem Paar 36 der Photodioden ein A/D-Wandler-Kanal zugeordnet. Die digitalisierten Signale werden zur Auswertung dem Prozessor 54 zugeleitet.

Es bleibt noch zu erwähnen, daß im Unterschied zur Ausführung der Figuren 1 und 2 im Ausführungsbeispiel gemäß Figur 3 die Paare 36 der Photodioden 30 bzw. der Flächenbereiche 22 nicht paarweise nebeneinander angeordnet sind, sondern sozusagen diametral und symmetrisch links bzw. rechts des ersten Bereichs 16 ausgebildet sind. Auch bei dieser Anordnung lassen sich mittels einer Differenzbildung der Ausgangssignale entsprechender Paare 36 von Photodioden 30 verbesserte Signal/Rauschverhältnisse bilden. Allerdings kann diese Anordnung, insbesondere bei Vorhandensein einer Vorzugsrichtung der Lichtausbreitung in dem Gewebe, von gewissem Nachteil sein.

Insoweit handelt es sich bei den dargestellten Ausführungsformen der Figur 1 und 2 bzgl. der paarigen Anordnung der Photodioden 30 bzw. weiteren Flächenbereiche 22 um eine bevorzugte Ausführungsform der Erfindung.

### Bezugszeichenliste

- 10 -: Vorrichtung
- 12 -: Gewebe
- 14 -: Lichtquelle
- 16 -: erster Bereich
- 18 -: Haut
- 20 -: Detektor
- 22 -: Flächenbereich
- 24 -: Lichtleitfaserstück
- 26 -: Kollimationsoptik
- 28 -: Lichtleitfaser
- 30 -: Photodiode
- 32 -: Polariationsfilter
- 34 -: Absorptionsfilter
- 36 -: Paar
- 38 -: Reihe
- 40 -: Gerade
- 42 -: Signal
- 44 -: Signal
- 46 -: Differenzverstärker
- 48 -: Vorverstärker
- 50 -: A/D-Wandler
- 52 -: Filterfunktion
- 54 -: Prozessor
- 56 -: Abstand
- 58 -: Meßkopf
- 60 -: Leiter
- 62 -: Füllmasse
- 64 -: Eindringtiefe
- 66 -: Photonenwegstrecke
- 68 -: Photonenwegstrecke
- 70 -: Kanal
- 72 -: Kanal
- 74 -: Verzögerungsstufe
- 76 -: Adaptionsstufe
- 78 -: Cepstrumanalysefunktion
- 80 -: Filterfunktion
- 82 -: A/D-Wandler
- 84 -: Zeitfenster
- 86 -: Fast-Fourier-Transformation
- 88 -: Leistungsspektrum
- 90 -: Logarithmierfunktion
- 92 -: inverse Fast-Fourier-Transformation
- 94 -: Lifterfunktion
- 96 -: Fast-Fourier-Transformation
- 98 -: mod. Leistungsspektrum
- 100 -: Längsachse

## Patentansprüche

1. Vorrichtung (10) zur tiefenselektiven, nichtinvasiven Messung von Muskelaktivitäten in menschlichem, tierischem oder dergleichen biologischem Gewebe (12), wobei eine kohärente, monochromatische Lichtquelle (14) zur Aussendung von Photonen hinein in das Gewebe (12) durch einen örtlich definierten ersten Bereich (16) des Gewebes (12) oder der das Gewebe (12) abdeckenden Haut (18) vorgesehen ist, und mehrere Detektoren (20) zur Erfassung der aus weiteren Flächenbereichen (22) der Haut (18) oder des Gewebes (12) wieder austretenden Photonen vorgesehen sind, wobei die weiteren Flächenbereiche in unterschiedlichen Abständen von dem ersten Bereich angeordnet sind, **dadurch gekennzeichnet, daß** die austretenden Photonen mittels der Detektoren (20) bezüglich Frequenz und Anzahl beziehungsweise Intensität detektiert werden und aus einem detektierten Interferenzmuster der aus dem jeweiligen Flächenbereich (22) und deren Abstand zum Einstrahlort austretenden Photonen in Verbindung mit frequenzunverschobenen Photonen eine tiefenselektive Detektion von Muskelaktivitäten durchgeführt wird.

2. Vorrichtung nach Anspruch 1, **dadurch gekennzeichnet, daß** der Lichtquelle (14) wenigstens ein dem Gewebe (12) stirnseitig aufbringbares oder aufsetzbares Lichtleitfaserstück (24) bzw. eine Kollimationsoptik (26) zur Fokussierung der Photonen auf dem Gewebe (12) bzw. der Haut (18) nachgeordnet ist.

3. Vorrichtung nach einem der vorhergehenden Ansprüche, **dadurch gekennzeichnet, daß** die Detektoren (20) jeweils eine dem Gewebe (42) stirnseitig aufbringbare oder aufsetzbare Lichtfaser (28) aufweisen, denen eine Photodiode (30) nachgeordnet ist.

4. Vorrichtung nach einem der vorhergehenden Ansprüche, **dadurch gekennzeichnet, daß** ein Polarisationsfilter (32) der Lichtquelle (14) nachgeschaltet und den Detektoren (20) vorgeschaltet ist, wobei den Detektoren (20) ein Absorptionsfilter (34) zugeordnet ist.

5. Vorrichtung nach einem der vorhergehenden Ansprüche, **dadurch gekennzeichnet, daß** die Wellenlänge der bevorzugt als Halbleiterlaser ausgebildeten Lichtquelle (14) im Bereich von 600 nm bis etwa 1200 nm, bevorzugt bei etwa 820 nm liegt.

6. Vorrichtung nach einem der vorhergehenden Ansprüche, **dadurch gekennzeichnet, daß** die weiteren Flächenbereiche (22) bzw. die zugeordneten Detektoren (20) paarweise einander benachbart und in Reihe (38) liegend hintereinander angeordnet sind, wobei jedes Paar (36) einen anderen Abstand zum ersten Bereich (16) aufweist und die Abstände benachbarter Paare (36) insbesondere äquidistant sind.

7. Vorrichtung nach einem der Ansprüche 1 bis 5, **dadurch gekennzeichnet, daß** die weiteren Flächenbereiche (22) bzw. die zugeordneten Detektoren (20) auf einer durch den ersten Bereich laufenden Geraden (40) und paarweise symmetrisch beidseitig des ersten Bereichs (16) angeordnet sind.

8. Vorrichtung nach einem der vorhergehenden Ansprüche, **dadurch gekennzeichnet, daß** die weiteren Flächenbereiche (22) bzw. die entsprechenden Detektoren (20) bis zu einem maximalen Abstand (56) von etwa 15 bis 30 mm von dem ersten Bereich (16) angeordnet sind.

9. Vorrichtung nach einem der vorhergehenden Ansprüche, **dadurch gekennzeichnet, daß** die von paarweisen Flächenbereichen (22) erfaßten beiden Signale (42, 44) jeweils den beiden Eingängen eines Differenzverstärkers (46) zugeführt werden.

10. Vorrichtung nach einem der vorhergehenden Ansprüche, **dadurch gekennzeichnet, daß** die Signale der Detektoren (20) unter anderem einer adaptiven Filterfunktion (52) sowie einer Cepstrumanalysefunktion (78) unterworfen werden.

11. Vorrichtung nach einem der vorhergehenden Ansprüche, **dadurch gekennzeichnet, daß** die Lichtquelle (14) sowie die Detektoren (20) und elektronische Komponenten, wie Vorverstärker (48), Differenzverstärker (46) oder Analog/Digital-Wandler (50) gemeinsam in einem Meßkopf (58), der flächig auf das Gewebe (12) bzw. die Haut (18) auflegbar ist, angeordnet sind, wobei der Meßkopf (58) lediglich mittels elektrischer Leiter (60) mit der Auswerteeinheit, insbesondere einem Prozessor (54), verbindbar ist.

12. Auswerteverfahren zur Messung von Muskelaktivitäten in menschlichem, tierischem oder dergleichen biologischem Gewebe (12), wobei man Photonen einer kohärenten, monochromatischen Lichtquelle (14) in das Gewebe (12) durch einen ersten Bereich (16) eintreten läßt, man in beabstandeten Flächenbereichen (22) aus dem Gewebe wieder austretende Photonen bezüglich Frequenz und Anzahl beziehungsweise Intensität detektiert, wobei die Flächenbereiche (22) in unterschiedlichen Abständen von dem ersten Bereich (16) angeordnet sind und man aus den Informationen Frequenz und Anzahl beziehungsweise Intensität in Verbindung mit dem jeweiligen Flächenbereich (22) als Austrittsort der wieder austretenden Photonen mittels eines Auswerteprogramms oder -algorithmus tiefenselektive Informationen der Muskelaktivität und/oder Anzahl der aktiven Muskeln und/oder räumlichen Lage der aktiven Muskeln im Gewebe (12) gewinnt.

13. Auswerteverfahren nach Anspruch 12, **dadurch gekennzeichnet, daß** man eine Lichtquelle (14) mit einer großen Kohärenzlänge größer als etwa 10 cm, verwendet, und man die jeweils in einem bestimmten Abstand von dem ersten Bereich (16) wieder austretenden Photonen, insbesondere zeitgleich wenigstens in zwei dicht benachbarten oder symmetrisch zum ersten Bereich (16) angeordneten Flächenbereichen (22) detektiert.

14. Auswerteverfahren nach Anspruch 13, **dadurch gekennzeichnet, daß** man die detektierten Signale jeweils zweier Flächenbereiche (22) unter anderem einer Differenzverstärkerfunktion (46) zuführt, sowie insbesondere einer adaptiven Filterfunktion (52) sowie einer Frequenz- oder Cepstrumanalysefunktion (78) unterwirft.

## Claims

1. Device (10) for deep-selective, non-invasive measurement of muscle activity in human, animal or the like biological tissue (12), wherein a coherent, monochromatic light source (14) for transmission of photons into the tissue (12) through a locally defined first region (16) of the tissue (12) or the skin (18) covering the tissue (12) is provided, and several detectors (20) for detection of the photons issuing back out of further surface regions (22) of the skin (18) or the tissue (12) are provided, wherein the further surface regions are arranged at different spacings from the first region, **characterised in that** the issuing photons are detected by means of the detectors (20) with respect to frequency and number or intensity and a deep-selective detection of muscle activity is undertaken from a detected interference pattern of the photons, which issue from the respective surface region (22) and the spacing thereof from the point of irradiation, in conjunction with photons non-displaced in frequency.

2. Device according to claim 1, **characterised in that** at least one optical conductor fibre member (24) able to be applied or placed at the end face on the tissue (12) or a collimation optical system (26) for focussing the photons on the tissue (12) or the skin (18) is arranged after the light source (14).

3. Device according to one of the preceding claims, **characterised in that** the detectors (20) each comprise an optical fibre (28) which is able to be applied or placed at the end face on the tissue (12) and downstream of which is arranged a photodiode (30).

4. Device according to one of the preceding claims, **characterised in that** a polarisation filter (32) is arranged downstream of the light source (14) and upstream of the detectors (20), wherein an adsorption filter (34) is associated with the detectors (20).

5. Device according to one of the preceding claims, **characterised in that** the wavelength of the light source (14), which is preferably constructed as a semiconductor laser, lies in the range of 600 nm to about 1200 nm, preferably at about 820 nm.

6. Device according to one of the preceding claims, **characterised in that** the further surface regions (22) or the associated detectors (20) are arranged adjacent to one another in pairs and lying one behind the other in rows (38), wherein each pair (36) has a different spacing from the first region (16) and the spacings of adjacent pairs (36) are, in particular, equidistant.

7. Device according to one of claims 1 to 5, **characterised in that** the further surface regions (22) or the associated detectors (20) are arranged on a straight line (40) running through the first region and in pairs symmetrically on both sides of the first region (16).

8. Device according to one of the preceding claims, **characterised in that** the further surface regions (22) or the associated detectors (20) are arranged up to a maximum spacing (56) of about 15 to 30 mm from the first region (16).

9. Device according to one of the preceding claims, **characterised in that** the two signals (42, 44) detected from the paired surface regions (22) are respectively fed to the two inputs of a differential amplifier.

10. Device according to one of the preceding claims, **characterised in that** the signals of the detectors (20) are subjected to inter alia an adaptive filter function (52) as well as a cepstrum analysis function (78).

11. Device according to one of the preceding claims, **characterised in that** the light source (14) as well as the detectors (20) and electronic components, such as pre-amplifier (48), differential amplifier (46) or analog-to-digital converter (50) are arranged in common in a measuring head (58) placeable flatly on the tissue (12) or the skin (18), wherein the measuring head (58) is connectible with the evaluating unit, especially a processor (54), merely by means of electrical conductors (60).

12. Evaluating method for measurement of muscle activity in human, animal or like biological tissue (12), wherein photons of a coherent monochromatic light source (14) are allowed to enter into the tissue (12) through a first region (16), photons issuing back out of the tissue in spaced surface regions (22) are detected with respect to frequency and number or intensity, wherein the surface regions (22) are arranged at different spacings from the first region (16), and deep-selective data of the muscle activity and/or number of active muscles and/or physical position of the active muscles in the tissue (12) are obtained by means of an evaluating program or algorithm from the frequency and number or intensity data in conjunction with the respective surface region (22) as exit location of the photons issuing back out.

13. Evaluating method according to claim 12, **characterised in that** a light source (14) with a large coherence length greater than approximately 10 cm is used and the photons, which issue again each time at a specific spacing from the first region (16), are detected, particularly at the same time, in at least two surface regions (22) which are closely adjacent or arranged symmetrically with respect to the first region (16).

14. Evaluating method according to claim 13, **characterised in that** the detected signals each time of two surface regions (22) are fed to inter alia a differential amplifier function (46) and in particular are subjected to an adaptive filter function (52) and to frequency or cepstrum analysis function (78).

## Revendications

1. Dispositif (10) de mesure sélective en profondeur, non-invasive, d'activités musculaires à l'intérieur de tissus humains, animaux, ou autres tissus biologiques analogues (12), dans lequel une source (14) de lumière cohérente monochromatique est prévue pour émettre des photons pénétrant dans le tissu (12) à travers une première zone locale définie (16) du tissu (12) ou de la peau (18) recouvrant le tissu (12), et dans lequel plusieurs détecteurs (20) sont prévus pour capter les photons ressortant à travers d'autres zones de surface (22) de la peau (18) ou du tissu (12), les autres zones de surface étant situées à différentes distances de la première zone, **caractérisé en ce que** les photons ressortants sont captés par les détecteurs (20) selon leur fréquence et leur nombre ou intensité, et **en ce que** l'on réalise, à partir d'un interférogramme détecté des photons ressortant de la zone de surface correspondante (22), et de la distance de celle-ci par rapport au point de radiation incidente, en se référant à des photons n'ayant subi aucun décalage de fréquence, une détection, sélective en profondeur, d'activités musculaires.

2. Dispositif selon la revendication 1, **caractérisé en ce qu'**en aval de la source de lumière (14) est disposé au moins un élément (24) à fibres optiques ou un système optique collimateur (26) pouvant être appliqué ou posé frontalement sur le tissu (12), permettant de focaliser les photons sur le tissu (12) ou la peau (18).

3. Dispositif selon l'une des revendications précédentes, **caractérisé en ce que** les détecteurs (20) comportent chacun une fibre optique (28) pouvant être appliquée ou posée frontalement sur le tissu (12), associée à une diode photoélectrique (30) montée en aval.

4. Dispositif selon l'une des revendications précédentes, **caractérisé en ce qu'**un filtre polarisant (32) est monté en aval de la source de lumière (14) et en amont des détecteurs (20), un filtre d'absorption (34) étant associé aux détecteurs (20).

5. Dispositif selon l'une des revendications précédentes, **caractérisé en ce que** la longueur d'onde de la source de lumière (14) de préférence réalisée sous forme d'un laser à semi-conducteur se situe dans la plage de 600 nm à environ 1200 nm, de préférence aux alentours de 820 nm.

6. Dispositif selon l'une des revendications précédentes, **caractérisé en ce que** les autres zones de surface (22) et les détecteurs associés (20) sont disposés par paires les uns à côté des autres et les uns derrière les autres en rangées (38), chaque paire (36) présentant une autre distance par rapport à la première zone (16), et l'espacement entre paires contiguës (36) étant notamment équidistant.

7. Dispositif selon l'une des revendications 1 à 5, **caractérisé en ce que** les autres zones de surface (22) et les détecteurs associés (20) sont disposés sur une droite (40) traversant la première zone, et par paires, de façon symétrique des deux côtés de la première zone (16).

8. Dispositif selon l'une des revendications précédentes, **caractérisé en ce que** les autres zones de surface (22) et les détecteurs associés (20) sont disposés jusqu'à une distance maximale (56) d'environ 15 à 30 mm de la première zone (16).

9. Dispositif selon l'une des revendications précédentes, **caractérisé en ce que** les deux signaux (42, 44) captés dans des zones de surface (22) d'une paire sont respectivement acheminés aux deux entrées d'un amplificateur différenciateur (46).

10. Dispositif selon l'une des revendications précédentes, **caractérisé en ce que** les signaux des détecteurs (20) sont soumis, entre autres, à une fonction adaptative de filtrage (52) ainsi qu'à une fonction d'analyse de cepstre (78).

11. Dispositif selon l'une des revendications précédentes, **caractérisé en ce que** la source de lumière (14) ainsi que les détecteurs (20) et des composants électroniques, tels qu'un préamplificateur (48), un amplificateur différenciateur (46) ou un convertisseur analogique/numérique (50), sont logés en commun dans une tête de mesure (58) qui peut être plaquée à plat sur le tissu (12) ou la peau (18), la tête de mesure (58) étant reliée à l'unité d'analyse, en particulier à un processeur (54), uniquement au moyen de conducteurs électriques (60).

12. Procédé d'analyse pour la mesure d'activités musculaires à l'intérieur de tissus humains, animaux, ou autres tissus biologiques (12) analogue, où l'on fait pénétrer des photons émis par une source (14) de lumière cohérente monochromatique dans le tissu (12), à travers une première zone (16), où l'on capte des photons ressortant du tissu dans différentes zones de surface (22) espacées de la première, en relevant leur fréquence et leur nombre ou intensité, les zones de surface (22) étant disposées à différentes distances de la première zone (16), et où l'on obtient, à partir des informations qui sont la fréquence et le nombre, voire l'intensité, associées à la zone de surface (22) constituant le point de sortie des photons ressortants, et grâce à un programme ou algorithme d'analyse, des informations sélectives, selon la profondeur, sur l'activité musculaire et/ou le nombre de muscles actifs et/ou la position dans l'espace des muscles actifs dans le tissu (12).

13. Procédé d'analyse selon la revendication 12, **caractérisé en ce que** l'on utilise une source de lumière (14) ayant une grande longueur de cohérence de plus de 10 cm environ, et **en ce que** l'on détecte les photons ressortant à une certaine distance de la première zone (16), en particulier ceux ressortant de façon simultanée dans au moins deux zones de surface (22) étroitement voisines ou disposées de façon symétrique par rapport à la première zone (16).

14. Procédé d'analyse selon la revendication 13, **caractérisé en ce que** les signaux détectés de deux zones de surface (22) contiguës sont soumis, entre autres, à une fonction d'amplificateur différenciateur (46) ainsi que, notamment, à une fonction adaptative de filtrage (52) ainsi qu'à une fonction (78) d'analyse de fréquence ou de cepstre.
